# EUROPÄISCHE PATENTSCHRIFT

(11) **EP 0 800 819 B1**
(45) Veröffentlichungstag und Bekanntmachung des Hinweises auf die Patenterteilung: **26.02.2003**
(21) Anmeldenummer: 97106262.5
(22) Anmeldetag: 16.04.1997
(51) Int. Cl.: A61K 7/48, A61K 7/50

(54) **Körperpflegemittel und Verfahren zu seiner Herstellung**
Skin and hair care composition and method of preparation
Composition pour le soin de la peau et des cheveux et procédé de préparation

(30) Priorität: 19.04.1996 DE 19615470
(43) Veröffentlichungstag der Anmeldung: 15.10.1997
(73) Patentinhaber: Beinio, Heinz, D-47551 Huisberden (DE)
(72) Erfinder: Beinio, Heinz, D-47551 Huisberden (DE)
(74) Vertreter: Bockhorni, Josef, Dipl.-Ing.

(56) Entgegenhaltungen:
- EP-A- 0 408 965
- WO-A-92/16216
- DE-A- 3 402 223
- DE-A- 19 523 459
- US-A- 5 489 395
- US-A- 5 494 675

## Beschreibung

Die Erfindung betrifft ein Körperpflegemittel, das insbesondere zur Pflege von Kopfhaut und Haaren bestimmt ist. Das Körperpflegemittel enthält eine besondere Abmischung aus Glykosiden und Schwarztorf als wasch- und pflegeaktive Komponente sowie ggf. übliche Zusätze.

Das erfindungemäße Pflegemittel ist besonders zur Pflege von Kopfhaut und Haaren geeignet, kann darüber hinaus aber allgemein als Badezusatz und Duschpflegemittel eingesetzt werden. Der darin enthaltende Schwarztorfzusatz hat eine Reihe von erwünschten Auswirkungen auf Haut und Haare. So bewirkt er eine schonende Desinfektion der Haut und des Haarbodens sowie eine Normalisierung der Talgdrüsenfunktion. Desweiteren kann damit einem Pilzbefall vorgebeugt und einem bestehenden Pilzbefall entgegengewirkt werden, so daß ein durch Pilzbefall bewirkter Haarausfall sowohl verhindert als auch behandelt werden kann.

WO-A-92 16216 und US-A-5 494 675 beschreiben Körperpflegemittel, die Torfextrakte enthalten.

Schwarztorf bildet den am stärksten zersetzten Teil des Moores, der zumeist unmittelbar über der wassertragenden Schicht angetroffen wird und häufig von sogenanntem Weißtorf überlagert ist. Aufgrund seiner Herkunft aus organischem Material weist dieses natürliche Substrat zahlreiche Inhaltsstoffe auf, beispielsweise Cellulose, Eiweißstoffe, Monound Disaccharide, Hemicellulosen, Pektinstoffe, Ligninstoffe, Fette, Bitumina, östrogene Stoffe, Gerbstoffe, Huminsäuren, Humine, Aminosäuren und Jod. Darüber hinaus sind zahlreiche anorganische Stoffe, beispielsweise Eisen, Schwefel in lipidlöslicher Form, Phosphate und Spurenelemente darin enthalten. Schwarztorf hat in der Regel einen pH-Wert von etwa 3,7; Werte unter 3,0 wurden aber ebenfalls gefunden.

Die Erfindung macht sich insbesondere die im Schwarztorf enthaltenden Konservierungsstoffe mit fungizider und antimyokotischer Wirkung zur Nutze. Schwarztorfabmischungen können lange Zeit unverändert aufbewart werden und zeigen vielfach auch nach Jahren noch keinen Ansatz von Schimmel oder geruchlicher Veränderung.

Die Erfindung macht sich ferner die physikalischen Wirkungen des Torfs zu Nutze, insbesondere sein Wärmeleitvermögen, seinen Einfluß auf den osmotischen Druck der Haut und die starke Sorption an der Haut. Durch die Permeabilität der Haut gelangen Ionen und Moleküle durch die Haut in den Körper, wodurch eine Veränderung des Mineralbestandes entsteht und außerdem eine Reizung der peripheren Nervenenden stattfindet. Es tritt eine vermehrte Durchblutung der Haut auf und die Funktionsmechanismen der Haut erfahren eine biologische Aktivierung.

Zu berücksichtigen ist allerdings, daß der niedrige pH-Wert des Schwarztorfs einer direkten Verwendung nicht in jedem Falle förderlich ist. Zwar sind sauer eingestellte Körperpflegemittel in der Praxis erprobt und als unbedenklich oder förderlich für die Hautpflege eingestuft, jedoch gilt dies nur für pH-Werte im Bereich von 5 bis 7. Die stark sauren pH-Werte des Schwarztorfs bedürfen daher der Abpufferung.

Diese Abpufferung wird auf vorteilhafter Weise dadurch erreicht, daß man dem erfindungsgemäßen Körperpflegemittel ein Glykosid als wasch- und reinigungsaktive Substanz zumischt. Das Gewichtsverhältnis von Schwarztorf zu Glykosid liegt dabei in einem Bereich von 25:75 bis 75:25, vorzugsweise bei 50:50. Da im Handel erhältliche Glykoside in der Regel stark alkalisch eingestellt sind - das Produkt Glukosid 24 der Hüls AG kommt als fünfzigprozentige wässrige Lösung mit einem pH-Wert von 9 bis 12 auf den Markt - ergibt sich hier eine vorteilhafte Ergänzung und ein im wesentlichen leicht sauer bis neutral eingestelltes Produkt. Im allgemeinen ist es zweckmäßig, die Abmischung so zu wählen, daß der pH-Wert des fertigen Pflegemittels im Bereich von 5 bis 7 und insbesondere bei 5,8 liegt.

Es hat sich gezeigt; daß Glykoside in Verbindung mit Schwarztorf eine ausgezeichnete Reinigungs- und Pflegewirkung entfalten. Bei der Anwendung bewirken die kleinen, kaum zerstörbaren Celluloseteilchen des Schwarztorfs nicht nur eine intensive Reinigung, sondern entfernen gleichzeitig der abgestorbenen Hornhaut durch einen sogenannten Peel-Effekt. Peel-Effekte werden in einer Reihe von Kosmetika durch Zusatz abrasiv wirkender Substanzen erzielt, beispielsweise von Polyurethanpulver. Die Erfindung beruht jedoch auf der Verwendung einer natürlichen Substanz, die weitere vorteilhafte physiologische Wirkungen hat und optimal mit einem Glykosid als weiterer wasch- und reinigungsaktiver Substanz zusammenwirkt.

Das erfindungsgemäße Körperpflegemittel enthält als wesentliche Bestandteile ein oder mehrere Glykoside und Schwarztorf in einem Gewichtsverhältnis von 25:75 bis 75:25 in wässriger Abmischung. Weitere Zusätze können zugegen sein, insbesondere solche, wie sie bei der Herstellung von Körperpflegemitteln verwandt werden. Zugesetzt werden können beispielsweise Geruchsstoffe, Hormone, Anti-Schuppen-Mittel, Haarpflegemittel, Farbstoffe und/oder andere in Pflegemitteln enthaltene Substanzen. Neben den Glykosiden können weitere waschaktive Substanzen zugegen sein. Zur Einstellung der gewünschten Konsistenz können viskositätregulierende Zusatzstoffe zugesetzt werden, desweiteren übliche Mittel zur Einstellung des pH-Wertes, sollte ein zuträglicher pH-Wert durch Abstimmung der Bestandteile Glykoside/Schwarztorf nicht erreichbar sein.

Vorzugsweise werden als Glykoside herrkömmliche Alkylpolyglukoside eingesetzt, die der allgemeinen Formel 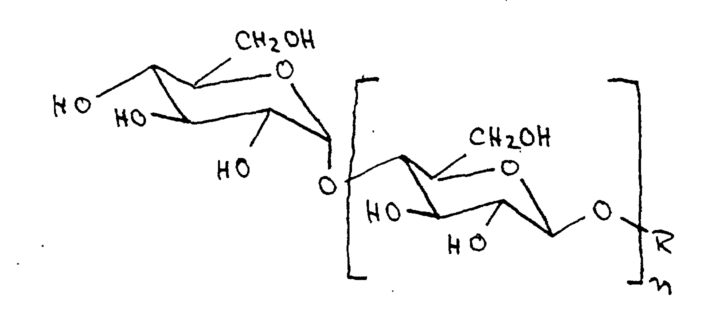 entsprechen, wobei n eine Zahl im Bereich von 1 bis 3 ist und R einen beliebigen Alkylrest, gradkettig oder verzweigt, mit bis zu 20 C-Atomen bezeichnet. Besonders bevorzugt ist ein Alkylpolyglukosid, bei dem n für eine Zahl von 1,2 bis 1,3 steht und R für einen Alkylrest mit 12 bis 14 Kohlenstoffatomen.

Ein solches Glukosid wird von der Firma Hüls AG unter der Bezeichnung Glukosid 24 vertrieben.

Neben den Glykosiden von Glukose können auch solche von Galaktose, Mannnose, Fructose und anderen Hexosen oder solche Pentosen, wie Arabinose, Xylose und Ribose eingesetzt werden. In Frage kommen neben Monoglykosiden auch Oligoglykoside mit wenigen, bis zu 3 Pyranose- oder Furanose-Einheiten.

Der erfindungsgemäß zum Einsatz kommende Schwarztorf liegt in homogenisierter und feinverteilter Form vor, vorzugsweise mit einer Teilchengröße von < 1,0 mm, wobei der überwiegende Teil der Schwarztorfteilchen im Bereich von 0,05 mm bis 0,5 mm liegen sollte. Die Verteilung und Homogenisierung des Schwarztorfs erfolgt zweckmäßigerweise durch kuttern.

Die erfindungsgemäßen Pflegemittel liegen in Form eines Gels oder einer Paste vor, wie herkömmliche Körperpflegemittel auch. Im allgemeinen kann die gewünschte Konsistenz durch Abmischung der Bestandteile mit Wasser erreicht werden; der Zusatz von Stellmittel ist aber ebenfalls möglich. Im allgemeinen erhalten die erfindungsgemäßen Körperpflegemittel 20 bis 50 Gew.-% Wasser und 25 bis 75 Gew.-% der Abmischung aus Schwarztorf und einem oder mehreren Glykosiden. Der pH-Wert der fertigen Abmischung sollte im Bereich von 5 bis 7 liegen, wobei ein Wert von 5,8 bevorzugt ist. Ein solcher Wert stellt sich im allgemeinen bei der Verwendung von gleichen Gewichtsteilen Schwarztorf und dem bevorzugten Glukosid der Fa. Hüls AG ein.

Die erfindungsgemäßen Körperpflegemittel werden nach einem speziell abgestimmten Verfahren hergestellt. Nach diesem Verfahren wird der Schwarztorf zerkleinert und zu einem Teig geknetet und homogenisiert. Diesem Teig wird während des Knetens und Homogenisierens das Glykosid zugesetzt, wobei ein leichtes Erwärmen notwendig sein kann, um das Glykosid über den Schmelzpunkt zu bringen. Die Temperatur der Knetmasse sollte aber nicht wesentlich über den Schmelzpunkt des Glykosids (10 bis 35°C bei dem Produkt der Hüls AG) erwärmt werden, um keine Aktivitätsverluste herbeizuführen. Nach Zugabe des Glykosids werden ggfs. übliche Zusätze eingemischt. Abschließend wird Wasser zur Einstellung der gewünschten Konsistenzen und Konzentration der Bestandteile zugesetzt.

Wie erwähnt, erfolgt das Zerkleinern des Schwarztorfs vorzugsweise durch kuttern. Dabei kann das Rohmaterial direkt gekuttert werden und anschließend zu einem Teig geknetet werden oder aber auch zuerst ein Teig bereitet werden, der als solcher dem Kutterprozeß unterworfen wird, bevor eine weitere Knet- und Homogenisierungsbehandlung erfolgt.

Das erfindungsgemäße Körperpflegemittel ist besonders als Pflegemittel für die Kopfhaut und die Haare geeignet. Der Peel-Effekt bewirkt eine zuverlässige Entfernung von Schuppen von der Kopfhaut und führt zu einem glatten Haar mit gutem Zusammenhalt und guter Geschmeidigkeit. Die nach der Verwendung vieler herkömmlicher Shampoos beobachteten "fliegenden" Haare wegen fehlenden Zusammenhalts der einzelnen Haare und elektrostatischer Aufladung kann zuverlässig vermieden werden. Gleichzeitig fördern die kleinen, auch nach der Zerkleinerung noch vorhandenen Celluloseteilchen die Durchblutung der Kopfhaut und fördern damit das Haarwachstum.

Es sei darauf hingewiesen, daß das erfindungsgemäße Körperpflegemittel ohne Einbuße von Wirksamkeit und Effektivität allein aus drei Komponenten nämlich Wasser, zerkleinertem Schwarztorf und der Glykosidkomponente hergestellt werden kann. Die beiden wasch- und reinigungsaktiven Zusätze sind dabei biologisch abbaubar, so daß eine Belastung des Wassers durch das Reinigungsmittel nicht eintritt. Soweit übliche Zusatzstoffe zugesetzt werden, zu denen auch Resorzin und Salicylate als Anti-Schuppen-Mittel sowie Hormone zur Förderung des Haarwachstums zählen, dienen diese lediglich zur Erweiterung des Wirkungsspektrums oder zur Erhöhung der Akzeptanz bei den angesprochenen Verkehrskreisen.

In der Praxis hat es sich bewährt, von unbehandelten Schwarztorf auszugehen, einer schwärzlichen, wasserhaltigen Masse aus weitgehend zersetzten Pflanzenresten. Bei Verwendung dieses Ausgangsstoffes wird diese Ausgangsmasse vor dem Homogenisieren zunächst durch Kneten in einen Teig umgearbeitet. Hierbei werden die unterschiedlich großen Partikel der Masse durchmischt und zum Teil bereits zerkleinert. Das Ausgangsprodukt läßt sich in einer Knetmaschine verarbeiten, wobei es erforderlich sein kann, die Teigmasse auf eine Temperatur geringfügig über den Schmelzpunkt der Glykosidkomponente zu erwärmen.

Dieser erster Verfahrensgang ermöglicht es, bereits beim Kneten dem Schwarztorf weitere Bestandteile des fertigen Pflegemittels zuzusetzen und dadurch das Anteigen zu vereinfachen und zu verbessern.

Der fertige Teig wird sodann homogenisiert, worunter eine intensive mechanische Einwirkung auf den Teig verstanden wird, die die Cellulosebestandteile soweit wie möglich zerkleinert. Dabei hat sich herausgestellt, daß ab einer bestimmten Größe die Celluloseteilchen nicht weiter zerkleinert werden, wodurch im fertigen Pflegemittel feine Schuppen überbleiben, die den oben erwähnten Peel-Effekt des Mittels ausmachen. Die Teilchengröße des Schwarztorfs wird durch Kuttern vorzugsweise des Teigs eingestellt. Ein Kuttern des unveränderten angelieferten Schwarztorfs ist aber ebenfalls möglich.

Beispielsweise hat sich die folgende Zusammensetzung für ein Haarpflegemittel gemäß der Erfindung als zweckmäßig herausgestellt:

| | |
|---|---|
| Schwarztorf | 35 Teile |
| Glukosid 24 der Fa. Hüls AG (Glukosidanteil) | 35 Teile |
| Wasser | 30 Teile |
| Parfüm q. s. | |

Alle angegebenen Teile sind Gewichtsteile. Das so erhaltene Haarpflegemittel hat einen pH-Wert von 5,8, einem für die Haut besonders gut verträglichen Wert. Die Konsistenz ist die eines streichfähigen, langsam fließenden Gels schwarzbrauner Farbe.

Durch entsprechende Variation der Bestandteile lassen sich mehr oder weniger Viskose Körperpflegemittel für verschiedene Zwecke herstellen.

Für den Fall, daß ein stärker alkalischer pH-Wert erfoderlich sein sollte, kann dies entweder durch Erhöhung des Glykosidzusatzes oder aber durch Verwendung einer Base, beispielsweise Triethanolamin, erreicht werden.

## Patentansprüche

1. Körperpflegemittel, insbesondere für Kopfhaut und Haare, auf Basis einer wässrigen Zubereitung, ggf. unter Verwendung üblicher Zusätze, **dadurch gekennzeichnet, daß** es eine Mischung aus einem oder mehreren Glykosiden und Schwarztorf in einem Gewichtsverhältnis von 25:75 bis 75:25 enthält.

2. Mittel nach Anspruch 1, **dadurch gekennzeichnet, daß** das Gewichtsverhältnis des oder der Glykoside zum Schwarztorf 50:50 beträgt.

3. Mittel nach Anspruch 1 oder 2, **dadurch gekennzeichnet, daß** als Glykosid ein Alkypolyglukosid der Formel 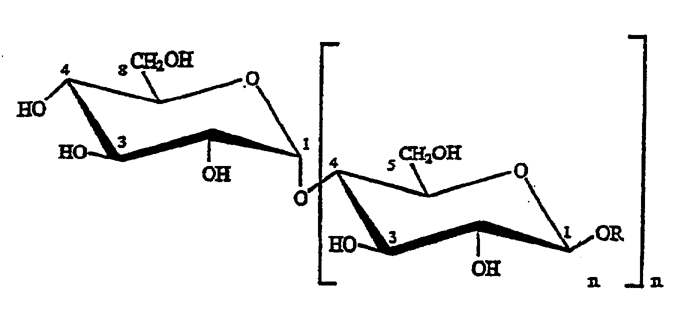 eingesetzt wird, wobei n für eine Zahl von 1 bis 3 steht und R einen Alkylrest mit bis zu 20 C-Atomen bezeichnet.

4. Mittel nach Anspruch 3, **dadurch gekennzeichnet, daß** n einen Wert von 1,2 bis 1,3 hat und R für einen Alkylrest mit 12 bis 14 C-Atomen steht.

5. Mittel nach einem der vorstehenden Ansprüche, **dadurch gekennzeichnet, daß** es den Schwarztorf in homogenisierter Form, gekuttert auf eine Teilchengröße < 1,0 mm, enthält.

6. Mittel nach einem der vorstehenden Ansprüche, **dadurch gekennzeichnet, daß** der Schwarztorf überwiegend eine Teilchengröße im Bereich von 0,05 mm bis 0,5 mm aufweist.

7. Mittel nach einem der vorstehenden Ansprüche in Form eines Gels oder einer Paste.

8. Mittel nach einem der vorstehenden Ansprüche, **dadurch gekennzeichnet, daß** es als weitere Zusätze Parfüm, Hormone und/oder Anti-Schuppen-Mittel enthält.

9. Mittel nach einem der vorstehenden Ansprüche, **dadurch gekennzeichnet, daß** es weitere waschaktive Substanzen enthält.

10. Mittel nach einem der vorstehenden Ansprüche, **dadurch gekennzeichnet, daß** es 25 bis 75 Gew.-% Glukosid/Schwarztorf und 20 bis 50 Gew.-% Wasser enthält.

11. Mittel nach einem der vorstehenden Ansprüche, **dadurch gekennzeichnet, daß** es auf ein pH-Wert von 5 bis 7 eingestellt ist.

12. Mittel nach Anspruch 11, **dadurch gekennzeichnet, daß** der pH-Wert 5,8 beträgt.

13. Verfahren zur Herstellung des Körperpflegemittels nach einem der vorstehenden Ansprüche, **dadurch gekennzeichnet, daß** Schwarztorf zerkleinert, zu einem Teig geknetet und homogenisiert wird, dem Glykosid und ggfs. übliche Zusätze zugemischt werden, wonach Wasser zur Einstellung der gewünschten Konsistenz zugesetzt wird.

14. Verfahren nach Anspruch 13, **dadurch gekennzeichnet, daß** der Schwarztorf auf eine Teilchengröße von < 1,0 mm gekuttert wird.

15. Verfahren nach Anspruch 13, **dadurch gekennzeichnet, daß** die Teilchengröße des Schwarztorfs so eingestellt wird, daß der überwiegende Teil der Schwarztorfteilchen im Bereich von 0,05 mm bis 0,5 mm vorliegt.

16. Verwendung des Körperpflegemittels nach einem der Ansprüche 1 bis 12 als Shampoo.

## Claims

1. Bodycare composition, particularly for the scalp and hair, based on an aqueous preparation, optionally with the use of conventional additives, **characterised in that** it contains a mixture of one or more glycosides and black peat in a weight ratio of 25:75 to 75:25.

2. Composition as claimed in Claim 1, **characterised in that** the weight ratio of the glycoside(s) to the black peat is 50:50.

3. Composition as claimed in Claim 1 or 2, **characterised in that** an alkypoly glucoside with the formula 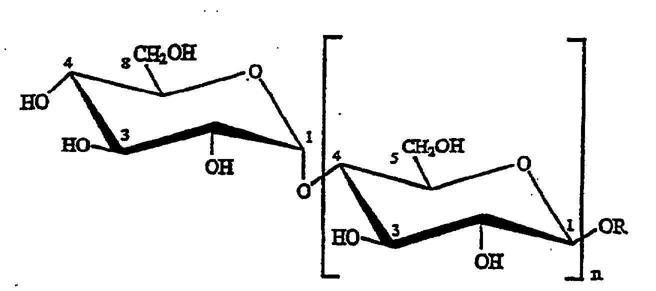 is used as the glycoside, n being a number from 1 to 3 and R designating an alkyl residue with up to 20 C atoms.

4. Composition as claimed in Claim 3, **characterised in that** n has a value of 1.2 to 1.3 and R is an alkyl residue with 12 to 14 C atoms.

5. Composition as claimed in one of the preceding claims, **characterised in that** it contains the black peat in homogenised form, cut to a particle size < 1.0 mm.

6. Composition as claimed in one of the preceding claims, **characterised in that** the black peat predominantly has a particle size in the range of 0.05 mm to 0.5 mm.

7. Composition as claimed in one of the preceding claims in the form of a gel or a paste.

8. Composition as claimed in one of the preceding claims, **characterised in that** it contains fragrance, hormones and/or an antidandruff agent as further additives.

9. Composition as claimed in one of the preceding claims, **characterised in that** it contains further washing active substances.

10. Composition as claimed in one of the preceding claims, **characterised in that** it contains 25 to 75% by wt. glucoside/black peat and 20 to 50% by wt. water.

11. Composition as claimed in one of the preceding claims, **characterised in that** it is adjusted to a pH value of 5 to 7.

12. Composition as claimed in Claim 11, **characterised in that** the pH value is 5.8.

13. Method of manufacturing the bodycare composition as claimed in one of the preceding claims, **characterised in that** black peat is comminuted, kneaded to form a paste and homogenised, and glycoside and optionally conventional additives are mixed in, whereafter water is added to obtain the desired consistency.

14. Method as claimed in Claim 13, **characterised in that** the black peat is cut to a particle size of < 1.0 mm.

15. Method as claimed in Claim 13, **characterised in that** the particle size of the black peat is so set that the predominant proportion of the black peat particles is present in the range of 0.05 mm to 0.5 mm.

16. Use of the bodycare composition as claimed in one of Claims 1 to 12 as a shampoo.

## Revendications

1. Composition de soins corporels, en particulier pour le cuir chevelu et les cheveux, à base d'une préparation aqueuse, le cas échéant avec utilisation d'additifs habituels, **caractérisée en ce qu'**elle contient un mélange d'un ou de plusieurs glycosides et de tourbe noire dans un rapport pondéral de 25:75 à 75:25.

2. Composition selon la revendication 1, **caractérisée en ce que** le rapport pondéral glycoside ou glycosides à tourbe noire est de 50:50.

3. Composition selon la revendication 1 ou 2, **caractérisée en ce qu'**on utilise comme glycoside un alkylpolyglycoside de formule 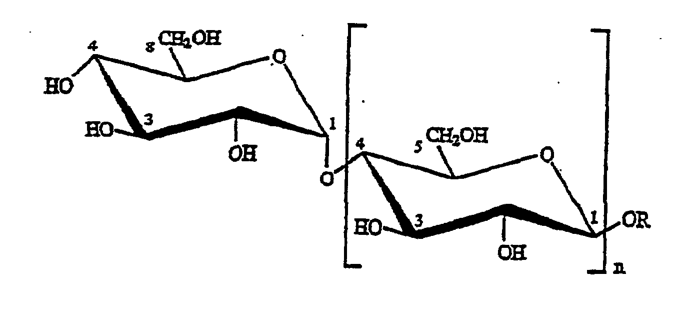 dans laquelle n est un nombre de 1 à 3 et R représente un radical alkyle avec jusqu'à 20 atomes de carbone.

4. Composition selon la revendication 3, **caractérisée en ce que** n a une valeur de 1,2 à 1,3 et R représente un radical alkyle avec de 12 à 14 atomes de carbone.

5. Composition selon l'une quelconque des revendications précédentes, **caractérisée en ce qu'**elle contient la tourbe noire sous forme homogénéisée, coupée à une taille de particules inférieure à 1,0 mm.

6. Composition selon l'une quelconque des revendications précédentes, **caractérisée en ce que** la tourbe noire présente principalement une taille de particules dans la plage de 0,05 mm à 0,5 mm.

7. Composition selon l'une quelconque des revendications précédentes sous forme d'un gel ou d'une pâte.

8. Composition selon l'une quelconque des revendications précédentes, **caractérisée en ce qu'**elle contient comme autres additifs du parfum, des hormones et/ou des agents antipelliculaires.

9. Composition selon l'une quelconque des revendications précédentes, **caractérisée en ce qu'**elle contient d'autres substances actives de lavage.

10. Composition selon l'une quelconque des revendications précédentes, **caractérisée en ce qu'**elle contient de 25 à 75 % en poids de glucoside/tourbe noire et de 20 à 50 % en poids d'eau.

11. Composition selon l'une quelconque des revendications précédentes, **caractérisée en ce qu'**elle est réglée à un pH de 5 à 7.

12. Composition selon la revendication 11, **caractérisée en ce que** le pH est de 5,8.

13. Procédé pour la préparation d'une composition de soins corporels selon l'une quelconque des revendications précédentes, **caractérisé en ce que** la tourbe noire est broyée, malaxée en une pâte et homogénéisée, **en ce que** le glycoside et, le cas échéant, des additifs usuels y sont mélangés, puis **en ce que** de l'eau y est ajoutée pour le réglage de la consistance souhaitée.

14. Procédé selon la revendication 13, **caractérisé en ce que** la tourbe noire est coupée à une taille de particules inférieure à 1,0 mm.

15. Procédé selon la revendication 13, **caractérisé en ce que** la taille de particules de la tourbe noire est réglée de telle manière que la partie principale des particules de tourbe noire soit située dans la plage de 0,05 mm à 0,5 mm.

16. Utilisation de la composition de soins corporels selon l'une quelconque des revendications 1 à 12 comme shampooing.
